# EUROPÄISCHE PATENTSCHRIFT

(11) **EP 0 373 463 B1**
(45) Veröffentlichungstag und Bekanntmachung des Hinweises auf die Patenterteilung: **18.01.1995**
(21) Anmeldenummer: 89122284.6
(22) Anmeldetag: 02.12.1989
(51) Int. Cl.: C07D 213/26, C07D 213/61

(54) **Verfahren zur Herstellung von 2-Chlor-5-chlormethylpyridin**
Process for the preparation of 2-chloro-5-chloromethyl pyridine
Procédé de préparation de pyridine-2-chloro-5-chlorométhyle

(30) Priorität: 16.12.1988 DE 3842358
(43) Veröffentlichungstag der Anmeldung: 20.06.1990
(73) Patentinhaber: BAYER AG, 51368 Leverkusen (DE)
(72) Erfinder: Jelich, Klaus, Dr., D-5600 Wuppertal 1 (DE)

(56) Entgegenhaltungen:
- EP-A- 0 072 777
- EP-A- 0 281 965
- DE-A- 2 814 330
- US-A- 2 695 902
- TETRAHEDRON LETTERS, Band 25, November 1984, Seiten 5693-5696, Oxford, GB; R.S.DAINTER et al.: "Abnormal nucleophilic substitution of 3-trichloromethylpyridines by methoxide"
- JOURNAL OF THE CHEMICAL SOCIETY, PERKIN TRANSACTIONS I, Februar 1989, Seiten 283-287, Cambridge; R.S. DAINTER et al.: "Transformations of trichloromethyl groups during reactions of 3-trichloromethylpyridines with methoxide"
- JOURNAL OF HETEROCYCLIC CHEMISTRY, Band 10, Oktober 1973, Seiten 779-784,Provo; S. NESNOW et al.: "Pyridine nucleosides related to 5-fluorouracil (1)"

## Beschreibung

Die Erfindung betrifft ein neues Verfahren zur Herstellung von 2-Chlor-5-chlormethylpyridin, welches als Zwischenprodukt zur Herstellung von bekannten Insektiziden verwendet wird.

Es ist bekannt, daß man 2-Chlor-5-chlormethylpyridin in einem aufwendigen mehrstufigen Verfahren erhält, wenn man 2-Chlorpyridin-5-carbonsäure mit Thionylchlorid in das entsprechende Säurechlorid überführt, dieses gegebenenfalls mit Ethanol verestert, anschließend mit Natriumboranat zur Hydroxymethylverbindung reduziert und schließlich mit Thionylchlorid in der Seitenkette die Hydroxygruppe durch Chlor substituiert (vgl. z. B. US-PS 4 576 629; J. Org. Chem. 34, 3545 [1969]; J. Heterocycl. Chem. 16, 333 - 337 [1979]).

Nachteilig bei diesem Verfahren und prohibitiv für eine großtechnische Durchführbarkeit ist jedoch der hohe Preis der Ausgangssverbindung 2-Chlorpyridin-5-carbonsäure und des Reduktionsmittels Natriumboranat, welches außerdem auch unter dem Aspekt der Wasserstofffreisetzung im Verlauf der Reaktion ein sicherheitstechnisches Problem darstellt.

Weiterhin ist bekannt, daß man 2-Chlor-5-chlormethylpyridin erhält, wenn man 2-Chlor-5-methylpyridin mit elementarem Chlor umsetzt (vgl. z. B. DE-A 36 30 046). Nachteilig bei diesem Verfahren ist jedoch, daß die Reaktion nicht einheitlich verläuft, so daß es nötig ist, um die Bildung von größeren Mengen an mehrfach chlorierten Nebenprodukten zu vermeiden, die Chlorierung frühzeitig abzubrechen, noch bevor die Umsetzung vollständig erfolgen konnte (vgl. auch EP-A 9 212; EP-A 65 358). Die entstehenden Produktgemische sind nur schwierig aufzutrennen und liefern Produkte mit nicht befriedigender Reinheit.

Es wurde nun gefunden, daß man 2-Chlor-5-chlormethylpyridin der Formel (I) 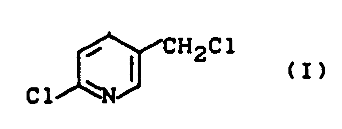
in hoher Ausbeute und großer Reinheit erhält, wenn man zunächst in einer 1. Stufe Nicotinsäure der Formel (II) 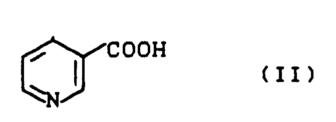
mit Phosphorpentachlorid in Gegenwart von Thionylchlorid und gegebenenfalls in Gegenwart eines Verdünnungmittels umsetzt,
dann in einer 2. Stufe das so erhältliche 3-Trichlormethylpyridin der Formel (III) 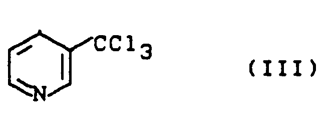
mit Alkalimetallalkoholaten der Formel (IV),

R - O - M (IV)

in welcher
- R: für Alkyl steht und
- M: für eine Alkalimetallkation steht,
gegebenenfalls in Gegenwart eines Verdünnungsmittels umsetzt,
dann in einer 3. Stufe die so erhältlichen Pyridinether-acetale der Formel (V), 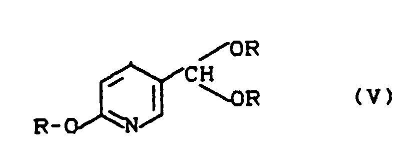
in welcher
- R: die oben angegebene Bedeutung hat,
mit verdünnter wäßriger Säure umsetzt,
dann in der 4. Stufe den so erhältlichen Pyridonaldehyd der Formel (VI) 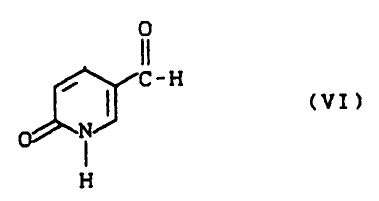
mit molekularem Wasserstoff in Gegenwart eines Hydrierkatalysators und gegebenenfalls in Gegenwart eines Verdünnungsmittels hydriert
und schließlich in einer 5. Stufe die so erhältliche Pyridylmethanolverbindung der Formel (VII) 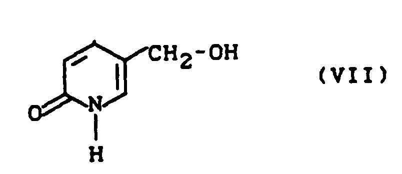
mit einem Chlorierungsmittel gegebenenfalls in Gegenwart eines Verdünnungsmittels und gegebenenfalls in Gegenwart eines Reaktionshilfsmittels umsetzt.

Die zweite und die dritte Stufe können ohne Isolierung der Zwischenprodukte direkt in einem Reaktionsschritt im Sinne eines sogenannten Eintopf-Verfahrens durchgeführt werden.

An der erfindungsgemäßen Reaktionsabfolge sind eine Reihe von Gesichtspunkten als völlig überraschend und für den Fachmann nicht vorhersehbar einzustufen. Weiterhin ist durch die Kombination der 5 Reaktionsstufen das Verfahren in seiner Gesamtheit neu und erfinderisch.

So konnte es beispielsweise nicht erwartet werden, daß die Umsetzung der Nicotinsäure mit Phosphorpentachlorid in Gegenwart von Thionylchlorid gemäß der ersten Stufe des erfindungsgemäßen Verfahrens in glatter Reaktion und sehr hohen Ausbeuten zu dem gewünschten 3-Trichlormethylpyridin der Formel (III) führen würde, da einerseits aus dem Stand der Technik bekannt war, daß zur Erzielung einer höheren Ausbeute sehr reaktionsfähige, aufwendig herzustellende und schwierig zu handhabende Phenylphosphinchloride als Chlorierungsmittel bzw. Reaktionshilfsmittel erforderlich seien (vgl. hierzu US-A 4 634 771) und da andererseits eigene Untersuchungen ergeben hatten, daß die einfache direkte Umsetzung von Nicotinsäure mit Phosphorpentachlorid (vgl. hierzu Tetrahedron Letters 25, 5693 - 5696 [1984]) in Substanz oder auch in Gegenwart von Verdünnungsmitteln nur Ausbeuten von maximal 5 % an gewünschter Trichlormethylpyridinverbindung der Formel (III) liefern.

Ebenfalls nicht vorhersehbar war die Tatsache, daß die Umsetzung von 5-Hydroxymethyl-2-pyridon der Formel (VII) mit Chlorierungsmitteln wie beispielsweise Phosphoroxychlorid oder Phosgen gemäß der fünften Stufe des erfindungsgemäßen Verfahrens zu einem gleichzeitigen Austausch sowohl der Hydroxygruppe in der Seitenkette als auch der Pyridonfunktion in der 2-Position des Pyridinsystems gegen jeweils einen Chlorrest führen würde. Eine derartige Reaktion war bisher aus dem Stand der Technik nicht bekannt.

Ganz besonders überraschend schließlich war die Tatsache, daß die Umsetzung der Pyridin-ether-acetale der Formel (V) mit verdünnten wäßrigen Säuren gemäß der dritten Stufe des erfindungsgemäßen Verfahrens nicht nur eine Spaltung der Acetalgruppierung in der Seitenkette bewirkt, sondern daß gleichzeitig auch unter ganz ungewöhnlich milden Bedingungen gleichzeiting eine Etherspaltung in der 2-Position des Pyridinringes stattfindet, da aus dem Stand der Technik bekannt war, daß ähnliche Etherspaltungen nur unter sehr viel drastischeren Reaktionsbedingungen, d. h. mit höher konzentrierten Säuren und bei höheren Temperaturen gelingen (vgl. z. B. Organikum, VEB Deutscher Verlag der Wissenschaften Berlin 1981; S. 237, 244, 245 oder J. Heterocycl. Chem. 10, 779, [1973]).

Als besonderer Vorteil der erfindungsgemäßen Reaktionssequenz muß herausgestellt werden, daß das Ausgangsprodukt Nicotinsäure ein billiges, großtechnisch herstellbares Ausgangsmaterial darstellt und daß darüberhinaus alle Umsetzungen mit leicht zugänglichen Reagenzien, unter technisch leicht herstellbaren Reaktionsbedingungen selektiv und in hoher Ausbeute durchführbar sind.

Verwendet man beispielsweise Nicotinsäure als Ausgangsverbindung, Thionylchlorid und Phosphorpentachlorid als Reaktionspartner in der ersten Stufe, Natriumisobutylat als Reagenz in der zweiten Stufe, verdünnte Salzsäure als Reagenz in der dritten Stufe, Raney-Nickel als Hydrierkatalysator in der vierten Stufe und Phosgen in Gegenwart von Dibutylformamid als Chlorierungsmittel in der fünften Stufe, so läßt sich der Reaktionsablauf des erfindungsgemäßen Verfahrens durch das folgende Formelschema darstellen:
1. Stufe: 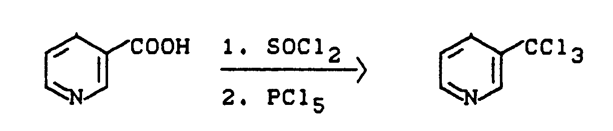
2. Stufe: 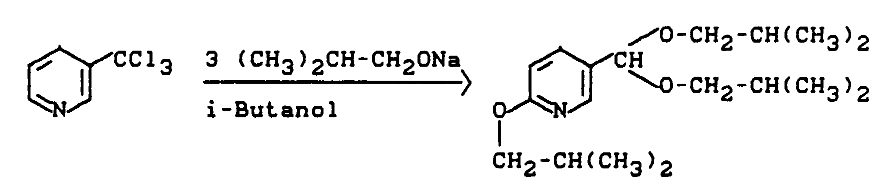
3. Stufe: 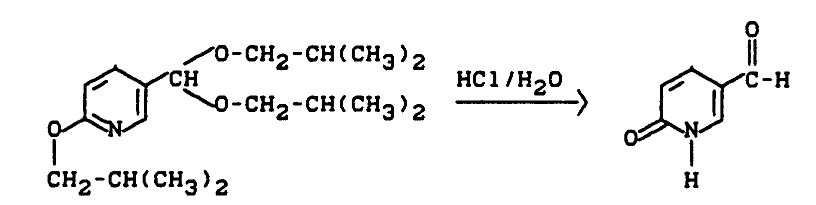
4. Stufe: 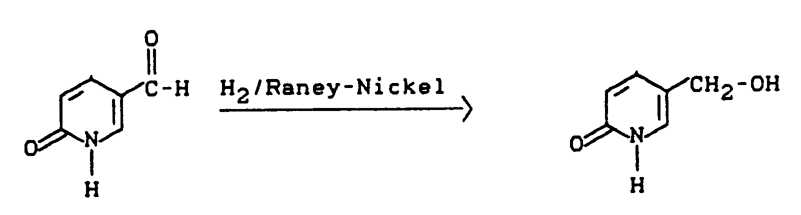
5. Stufe: 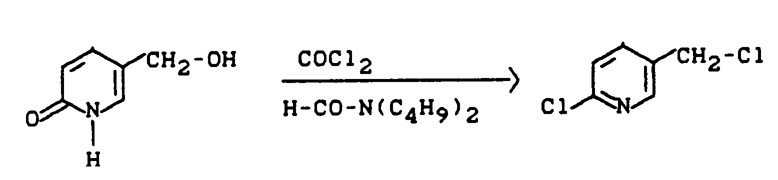
Als Verdünnungsmittel zur Durchführung der ersten Stufe des erfindungsgemäßen Verfahrens kommen inerte organische Lösungsmittel in Frage. Mit besonderem Vorzug verwendet man Benzol, Toluol, Xylol, Chlorbenzol, Dichlorbenzol, Nitrobenzol oder Phosphoroxychlorid.
Es ist auch möglich, die erste Stufe des erfindungsgemäßen Verfahrens ohne Verwendung eines Verdünnungsmittels direkt in Substanz durchzuführen.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen 80 °C und 180 °C, vorzugsweise bei Temperaturen zwischen 110 °C und 160 °C.

Zur Durchführung der ersten Stufe des erfindungsgemäßen Verfahrens setzt man pro Mol an Nicotinsäure der Formel (II) entweder 2 bis 4 Mol an Phosphorpentachlorid oder zur Vermeidung eines größeren Phosphorpentachloridüberschusses zunächst 1 bis 2 Mol Thionylchlorid (hierbei wird das Ausgangsprodukt Nicotinsäure in das Hydrochlorid des entsprechenden Säurechlorids überführt) und anschließend 1 bis 2 Mol an Phosphorpentachlorid ein. Es ist auch möglich, das Phosphorpentachlorid aus einer entsprechenden Menge Phosphortrichlorid und einer equivalenten Menge Chlor direkt im Reaktionsgefäß herzustellen. Im Verlauf der Reaktion erweist es sich als vorteilhaft, freiwerdendes Phosphoroxychlorid kontinuierlich abzudestillieren.

Die Aufarbeitung und Isolierung des Reaktionsproduktes der Formel (III) kann durch Destillation erfolgen; das Rohprodukt kann aber auch direkt für die weitere Umsetzungen eingesetzt werden.

Die zur Durchführung der zweiten Stufe des erfindungsgemäßen Verfahrens als Ausgangsstoffe benötigten Alkalimetallalkoholate sind durch die Formel (IV) allgemein definiert. In dieser Formel (IV) steht R vorzugsweise für geradkettiges oder verzweigtes Alkyl mit 1 bis 4 Kohlenstoffatomen, insbesondere für Methyl, Ethyl, Isopropyl, i-Butyl oder sec-Butyl.
M steht vorzugsweise für ein Natrium- oder Kaliumkation, insbesondere für Natrium.

Die Alkalimetallalkoholate der Formel (IV) sind allgemein bekannte Verbindungen; sie können gegebenenfalls in situ aus Alkalimetallhydroxiden und entsprechenden Alkoholen erzeugt werden.

Als Verdünnungsmittel zur Durchführung der zweiten Stufe des erfindungsgemäßen Verfahrens kommen ebenfalls inerte organische Lösungsmittel in Frage. Mit besonderem Vorzug verwendet man niedere Alkylalkohole, welche den gleichen Alkylrest tragen, durch den die als Reaktionspartner verwendeten Alkalimetallalkoholate der Formel (IV) gekennzeichnet sind, insbesondere Methanol, Ethanol, Isopropanol oder Isobutanol.

Die Reaktionstemperaturen können bei der Durchführung der zweiten Stufe des erfindungsgemäßen Verfahrens in einem größeren Bereich variiert werden. Im allgemeinen arbeitet man bei Temperaturen zwischen 0 °C und 120 °C, vorzugsweise bei Temperaturen zwischen 20 °C und 90 °C.

Zur Durchführung der zweiten Stufe des erfindungsgemäßen Verfahrens setzt man pro Mol an 3-Trichlormethylpyridin der Formel (III) im allgemeinen 3.0 bis 15.0 Mol, vorzugsweise 3.5 bis 6.0 Mol an Alkalimetallalkoholat der Formel (IV) ein.
Die Reaktionsdurchführung, Aufarbeitung und Isolierung der Reaktionsprodukte erfolgt nach üblichen Methoden (vgl. die Herstellungsbeispiele).

Als Säuren zur Durchführung der dritten Stufe des erfindungsgemäßen Verfahrens kommen vorzugsweise verdünnte organische oder anorganische Protonensäuren in Frage. Vorzugsweise verwendet man verdünnte wäßrige Salzsäure, Essigsäure, Ameisensäure oder Schwefelsäure als Reaktionsmedium.

Die Reaktionstemperaturen können bei der Durchführung der dritten Stufe des erfindungsgemäßen Verfahrens in einem größeren Bereich variiert werden. Im allgemeinen arbeitet man bei Temperaturen zwischen 20 °C und 120 °C, vorzugsweise bei Temperaturen zwischen 50 °C und 100 °C.

Zur Durchführung der dritten Stufe des erfindungsgemäßen Verfahrens setzt man die Pyridin-ether-acetale der Formel (V) im allgemeinen in einer 0.2 bis 5.0 %igen, insbesondere in einer 0,5 bis 1.0 %igen wäßrigen Säurelösung bei der erforderlichen Reaktionstemperatur um. Die Aufarbeitung und Isolierung der Reaktionsprodukte erfolgt nach allgemein üblichen Methoden (vgl. auch die Herstellungsbeispiele).

Als Hydrierkatalysatoren zur Durchführung der 4. Stufe des erfindungsgemäßen Verfahrens kommen übliche Edelmetall-, Edelmetalloxid- oder Raney-Katalysatoren, gegebenenfalls auf einem geeigneten Träger, wie beispielsweise Aktivkohle, Aluminiumoxid oder Siliciumdioxid in Frage. Mit besonderem Vorteil verwendet man Palladium auf Aktivkohle oder Raney-Nickel.

Als Verdünnungsmittel zur Durchführung der vierten Stufe des erfindungsgemäßen Verfahrens kommen inerte organische Lösungsmittel in Frage. Hierzu gehören insbesondere Ether wie Diethylether, Dioxan, Tetrahydrofuran oder Ethylglykoldimethyl- oder- diethylether, Alkohole wie Methanol, Ethanol, Propanol, Butanol, Ethylenglykolmonomethylether oder Ethylenglykolmonoethylether oder Säuren wie beispielsweise Essigsäure.

Die Reaktionstemperaturen können bei der Durchführung der vierten Stufe des erfindungsgemäßen Verfahrens in einem größeren Bereich variiert werden. Im allgemeinen arbeitet man bei Temperaturen zwischen 0 °C und 150 °C, vorzugsweise bei Temperaturen zwischen 20 °C und 100 °C.

Die vierte Stufe des erfindungsgemäßen Verfahrens wird vorzugsweise unter erhöhtem Druck durchgeführt. Im allgemeinen arbeitet man in Druckbereichen zwischen 2 und 200 bar, vorzugsweise zwischen 10 und 100 bar.

Zur Durchführung der vierten Stufe des erfindungsgemäßen Verfahrens setzt man pro Mol an Pyridonaldehyd der Formel (VI) im allgemeinen 1.0 bis 20.0 Mol, vorzugsweise 1.0 bis 5.0 Mol an Wasserstoff und 0.0001 bis 1.0 Mol, vorzugsweise 0.01 bis 0.1 Mol an Hydrierkatalysator ein.

Die Reaktionsdurchführung, Aufarbeitung und Isolierung der Reaktionsprodukte erfolgt nach allgemein üblichen Methoden (vgl. auch die Herstellungsbeispiele).

Als Chlorierungsmittel zu Durchführung der fünften Stufe des erfindungsgemäßen Verfahren kommen insbesondere Phosphorpentachlorid, Phsophoroxychlorid oder Phosgen, sowie auch Gemische dieser Verbindungen in Frage.

Die fünfte Stufe des erfindungsgemäßen Verfahrens kann entweder ohne Zusatz eines Verdünnungmittels direkt in Substanz oder in Gegenwart eines geeigneten Verdünnungsmittels duchgeführt werden. Hierzu gehören insbesondere aliphatische, alicyclische oder aromatische, gegebenenfalls halogenierte Kohlenwasserstoffe, wie beispielsweise Benzol, Toluol, Xylol, Chlorbenzol oder Dichlorbenzol, Petrolether, Hexan, Cyclohexan, Methylcyclohexan, Dichlormethan, Chloroform oder Tetrachlorkohlenstoff.

Die fünfte Stufe des erfindungsgemäßen Verfahrens kann gegebenenfalls in Gegenwart eines geeigneten Reaktionshilfsmittels durchgeführt werden. Als solche kommen tertiäre Amine, wie Triethylamin, N,N-Dimethylanilin, Pyridin oder N,N-Dimethylaminopyridin in Frage, außerdem auch katalytische Mengen an Formamiden wie beispielsweise N,N-Dimethylformamid oder N,N-Dibutylformamid oder anorganische Metallchloride wie Magnesiumchlorid oder Lithiumchlorid.

Die Reaktionstemperaturen können bei der Durchführung der fünften Stufe des erfindungsgemäßen Verfahrens in einem größeren Bereich variiert werden. Im allgemeinen arbeitet man bei Temperaturen zwischen 20 °C und 200 °C, vorzugsweise bei Temperaturen zwischen 60 °C und 150 °C.

Zur Durchführung der fünften Stufe des erfindungsgemäßen Verfahrens setzt man pro Mol an Pyridylmethanol der Formel (VII) im allgemeinen 1.0 bis 10.0 Mol, vorzugsweise 1.0 bis 5.0 Mol an Chlorierungsmittel und gegebenenfalls 0.01 bis 3.0 Mol, vorzugsweise 0.1 bis 2.0 Mol an Reaktionshilfsmittel ein.

Die Reaktionsdurchführung, Aufarbeitung und Isolierung des Reaktionsproduktes der Formel (I) erfolgt mit Hilfe bekannter Verfahren (vgl. auch die Herstellungsbeispiele).
Die mit Hilfe des erfindungsgemäßen Verfahrens erhältliche Verbindung 2-Chlor-5-chlormethylpyridin der Formel (I) ist eine bekannte Verbindung und kann beispielsweise als Zwischenprodukt für die Herstellung von insektiziden Nitromethylenverbindungen verwendet werden (vgl. z. B. EP-A 163 855; EP-A 192 060; EP-A 259 738; EP-A 254 859).

### Herstellungsbeispiele

### 1. Stufe:

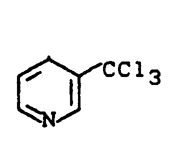

Zu 250 ml Thionylchlorid gibt man im Verlauf von 10 Minuten 123,1 g (1 Mol) Nicotinsäure, wobei sich die Mischung auf 50 °C erwärmt. Nach beendeter Zugabe rührt man 15 Minuten bei 55 °C und destilliert dann überschüssiges Thionylchlorid unter vermindertem Druck ab. Danach gibt man 275 g (2 Mol) Phosphortrichlorid zu und leitet im Verlauf von 2 Stunden insgesamt 140 g (2 Mol) getrocknetes Chlorgas ein, wobei sich die Mischung auf 70 °C erwärmt. Danach erhitzt man für eine Stunde auf 150 °C, wobei freiwerdendes Phosphoroxychlorid kontinuierlich abdestilliert wird. Zur Aufarbeitung wird abgekühlt, 600 ml Essigester zugegeben, die Mischung in Eiswasser gegossen, durch portionsweise Zugabe von Natriumcarbonat unter Kühlen schwach alkalisch gestellt, die organische Phase abgetrennt und die wäßrige Phase mit 300 ml Essigester extrahiert. Die vereinigten organischen Phasen werden über Magnesiumsulfat getrocknet, im Vakuum eingeengt und im Wasserstrahlvakuum destilliert.

Man erhält 176,8 g (89 % der Theorie) an 3-Trichlormethylpyridin vom Siedepunkt 105 °C - 107 °C bei 15 mbar.

### 2. Stufe:

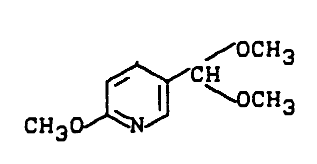

Zu 140,7 g (0,515 Mol) einer Lösung von Natriummethylat in Methanol gibt man bei Rückflußtemperatur tropfenweise unter Rühren im Verlauf von 45 Minuten 31,6 g (0,161 Mol) 3-Trichlormethylpyridin. Nach beendeter Zugabe rührt man weitere 3 Stunden bei Rückflußtemperatur, kühlt dann ab, filtriert, engt das Filtrat ein, nimmt den Rückstand in Dichlormethan auf, filtriert ein weiteres Mal, engt das Filtrat ein und destilliert den Rückstand im Vakuum.

Man erhält 24,5 g (83% der Theorie) an 2-Methoxy-5-bis-(methoxy)methyl-pyridin vom Siedepunkt 54 °C - 55 °C bei 0,25 mbar.

### 3. Stufe:

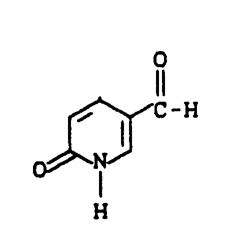

41,5 g (0,227 Mol) 2-Methoxy-5-bis-(methoxy)-methyl-pyridin werden in 300 ml 0,5 %iger wäßriger Salzsäure 3 Stunden auf Rückflußtemperatur erhitzt, anschließend eingeengt bis auf ein Volumen von ca. 120 ml, auf 0 °C abgekühlt, ausgefallener Feststoff abgesaugt und getrocknet.

Man erhält 22.5 g (81 % der Theorie) an 2-Pyridinon-5-aldehyd vom Schmelzpunkt 222 °C.

### 4. Stufe

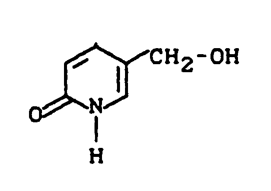

Zu 10,0 g (0,081 Mol) 2-Pyridinon-5-aldehyd in 200 ml Ethanol gibt man 2 g Raney-Nickel und hydriert anschließend 4 Stunden bei 80 °C und 30 bar Wasserstoffdruck. Zur Aufarbeitung wird der Katalysator abfiltriert, das Filtrat eingeengt und der zurückbleibende Feststoff durch Verrühren mit Essigester, Absaugen und Trocknen gereinigt.

Man erhält 7 g (78 % der Theorie) an 5-Hydroxymethyl-2-pyridinon vom Schmelzpunkt 130 °C.

### 5. Stufe

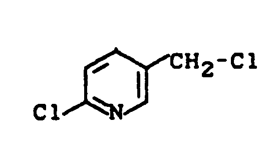

Zu einer Mischung von 6,7 g (0,032 Mol) Phosphorpentachlorid und 2,5 g (0,016 Mol) Phosphoroxychlorid gibt man 2,0 g (0,016 Mol) 5-Hydroxymethyl-2-pyridinon, rührt 7 Stunden bei Rückflußtemperatur, kühlt ab, nimmt die Mischung in Essigester auf, gibt dann Eiswasser zu, neutralisiert mit Natriumcarbonat, trennt die organische Phase ab, trocknet über Magnesiumsulfat und entfernt das Lösungsmittel unter vermindertem Druck. Der Rückstand läßt sich destillativ reinigen.

Man erhält 2,5 g (96 % der Theorie) an 2-Chlor-5-chlormethylpyridin vom Siedepunkt 70 °C - 80 °C bei 1 mbar.

## Patentansprüche

1. Verfahren zur Herstellung von 2-Chlor-5-chlormethyl-pyridin der Formel (I) 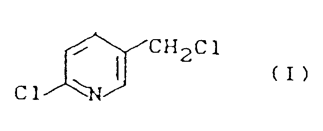 dadurch gekennzeichnet, daß man Nicotinsäure der Formel (II) 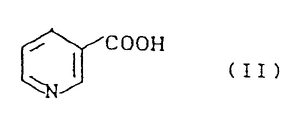 mit Phosporpentachlorid in Gegenwart von Thionylchlorid und gegebenenfalls in Gegenwart eines inerten organischen Lösungsmittels umsetzt,
dann in einer 2. Stufe das so erhältliche 3-Trichlormethylpyridin der Formel (III) 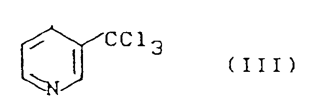 mit Alkalimetallalkoholaten der Formel (IV),
R - O - M (IV)
in welcher
in welcher
R für Alkyl steht und
M für eine Alkalimetallkation steht,
gegebenenfalls in Gegenwart eines Verdünnungsmittels umsetzt,
dann in einer 3. Stufe die so erhältlichen Pyridinether-acetale der Formel (V), 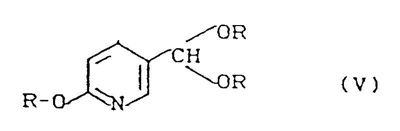 in welcher
R die oben angegebene Bedeutung hat,
mit verdünnter wäßriger Säure umsetzt,
dann in der 4. Stufe den so erhältlichen Pyridonaldehyd der Formel (VI) 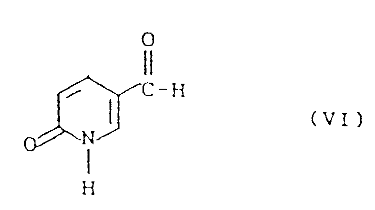 mit molekularem Wasserstoff in Gegenwart eines Hydrierkatalysators und gegebenenfalls in Gegenwart eines Verdünnungsmittels hydriert
und schließlich in einer 5. Stufe die so erhältliche Pyridylmethanolverbindung der Formel (VII) 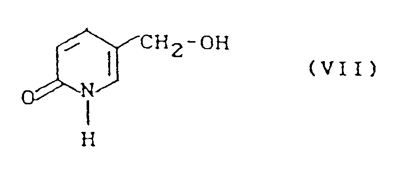 mit einem Chlorierungsmittel gegebenenfalls in Gegenwart von aliphatischen, alicyclischen, aromatischen gegebenenfalls halogenierten Kohlenwasserstoffen als Verdünnungsmittel und in Gegenwart von tertiären Aminen, anorganischen Metallchloriden oder katalytischen Mengen an Formamiden umsetzt.

2. Verfahren gemäß Anspruch 1, dadurch gekennzeichnet, daß man die zweite und die dritte Stufe ohne Isolierung der Zwischenprodukte durchführt.

## Claims

1. Process for the preparation of 2-chloro-5-chloromethyl-pyridine, of the formula (I), 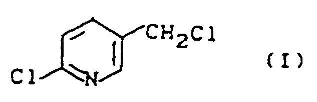 characterized in that nicotinic acid, of the formula (II), 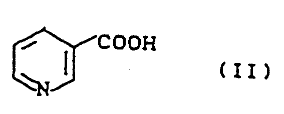 is reacted with phosphorus pentachloride in the presence of thionyl chloride and if appropriate in the presence of an inert organic solvent,
the resulting 3-trichloromethylpyridine, of the formula (III), 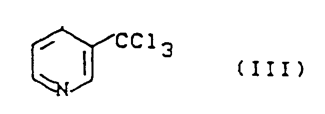 is then reacted, in a 2nd step, with alkali metal alkoxides of the formula (IV)
R - O - M (IV)
in which
R represents alkyl and
M represents an alkali metal cation,
if appropriate in the presence of a diluent,
the resulting pyridine ether acetals of the formula (V) 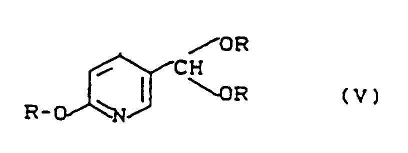 in which
R has the abovementioned meaning,
are then reacted in a 3rd step with dilute aqueous acid,
the resulting pyridone aldehyde, of the formula (VI), 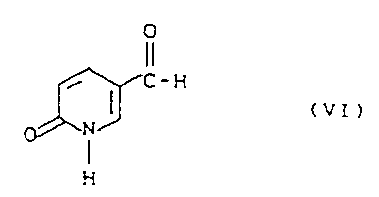 is then hydrogenated in the 4th step with molecular hydrogen in the presence of a hydrogenation catalyst and if appropriate in the presence of a diluent and the resulting pyridylmethanol compound, of the formula (VII), 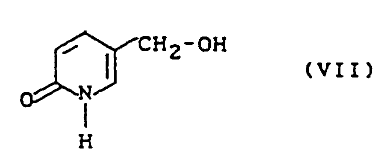 is finally reacted in a 5th step with a chlorinating agent, if appropriate in the presence of aliphatic, alicyclic, aromatic, optionally halogenated hydrocarbons as diluent and in the presence of tertiary amines, inorganic metal chlorides or catalytic amounts of formamides.

2. Process according to Claim 1, characterized in that steps two and three are carried out without isolation of the intermediates.

## Revendications

1. Procédé de préparation de 2-chloro-5-chlorométhylpyridine de formule (I) 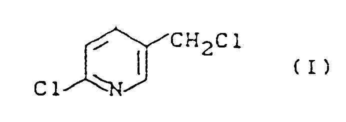 caractérisé en ce que l'on fait réagir l'acide nicotinique de formule (II) 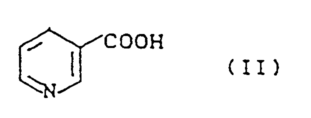 avec le pentachlorure de phosphore en présence de chlorure de thionyle et, éventuellement, en présence d'un solvant inerte organique puis,
au cours d'une deuxième étape, on fait réagir la 3-trichlorométhylpyridine de formule (III) ainsi obtenue 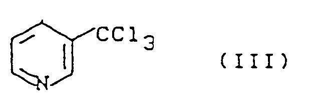 avec des alcoolates des métaux alcalins de formule (IV)
R - O - M (IV)
dans laquelle
R représente un radical alkyle et
M représente un cation de métal alcalin,
éventuellement en présence d'un diluant puis, au cours d'une troisième étape, on fait réagir l'étheracétal de pyridine de formule (V) ainsi obtenu 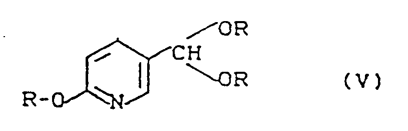 dans laquelle
R a la signification précitée,
avec un acide en solution diluée puis, au cours d'une quatrième étape, l'aldéhyde pyridonique de formule (VI) ainsi obtenu 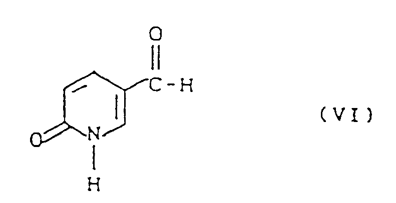 est hydrogéné par l'hydrogène moléculaire en présence d'un catalyseur d'hydrogénation et, éventuellement, en présence d'un diluant,
et que, enfin, au cours d'une cinquième étape, le composé de pyridylméthanol ainsi obtenu de formule (VII) 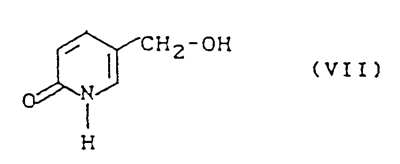 est mis à réagir avec un agent de chloration , éventuellement en présence d'hydrocarbures aliphatiques, alicycliques, aromatiques éventuellement halogénés servant de diluant et en présence d'amines tertiaires, de chlorures métalliques inorganiques ou de quantités catalytiques de formamides.

2. Procédé selon la revendication 1,
caractérisé en ce que la deuxième et la troisième étape sont réalisées sans séparation des produits intermédiaires.
